# DEMANDE DE BREVET EUROPEEN

(11) **EP 1 050 284 A1**
(43) Date de publication de la demande: **08.11.2000**
(21) Numéro de dépôt: 99420115.0
(22) Date de dépôt: 07.05.1999
(51) Int. Cl.: A61F 2/42

(54) **Prothèse d'articulation métacarpo-phalangienne ou inter-phalangienne**

(71) Demandeur: FIXANO SA, F-01000 Bourg en Bresse (FR)
(72) Inventeur: Regnard, Pierre-Jean, 21800 Chevigny St. Sauveur (FR); Sartorius, Christian, 38240 Meylan (FR); Lemarechal, Pierre, 57100 Thionville (FR); Martin, Jean-Jacques, 01000 Bourg en Bresse (FR)
(74) Mandataire: Bratel, Gérard

(57) **Abrégé**

Cette prothèse comprend deux éléments (2, 3, 10 à 12) destinés à être ancrés respectivement dans les canaux médullaires des os concernés, dont un (4, 5) présente une surface condylienne (7) et l'autre (10 à 12) une surface glénoïde (14).

Selon l'invention :
- ladite surface condylienne (7) a un rayon de courbure qui augmente en direction de la partie palmaire de cette surface (7), de sorte que cette dernière présente la forme d'une portion de surface sphérique aplatie ;
- l'élément (2, 3) comprend un doigt allongé pivotant (21), ce doigt (21) pouvant pivoter entre une position parallèle à l'axe longitudinal dudit élément (2, 3) et une position perpendiculaire à cet axe, et
- l'élément (10 à 12) présentant la surface glénoïde (14) comprend une cavité axiale (25) dans laquelle ce doigt (21) est engagé et peut coulisser.

## Description

La présente invention concerne une prothèse d'articulation métacarpo-phalangienne ou inter-phalangienne.

Un type de prothèse connu pour appareiller une telle articulation comprend deux tiges destinées à être insérées dans les canaux médullaires des deux os concernés, qui sont reliées l'une à l'autre par une partie souple formant charnière, notamment en silicone.

Ce type de prothèse ne donne pas parfaitement satisfaction en termes de reproduction du mouvement anatomique, du contrôle de la laxité latérale ou axiale de l'articulation, et de la résistance de la prothèse dans le temps.

Un autre type de prothèse connu pour appareiller ce type d'articulation comprend deux éléments ancrés respectivement, au moyen de tiges d'ancrage, dans les os concernés, dont un comprend une surface condylienne et l'autre une surface glénoïde.

La résistance à l'usure d'une prothèse de ce type est améliorée, mais le contrôle de la laxité ou la reproduction du mouvement anatomique ne sont pas parfaits.

La présente invention vise à remédier à ces inconvénients essentiels.

La prothèse qu'elle concerne comprend, de manière connue en soi, deux éléments destinés à être ancrés respectivement, au moyen de tiges d'ancrage, dans les canaux médullaires des os concernés, dont un présente une surface condylienne et l'autre une surface glénoïde.

Selon l'invention :
- ladite surface condylienne a une forme courbe mais avec un rayon de courbure non constant, qui augmente en direction de l'extrémité axiale de cette surface située du côté opposé à la tige d'ancrage, de sorte que cette surface présente la forme d'une portion de sphère aplatie, ladite extrémité axiale étant "palmaire", c'est-à-dire présentant un relativement faible rayon de courbure ;
- l'élément comprenant cette surface condylienne comprend, au niveau de sa partie qui forme cette même surface, une cavité centrale délimitée par deux faces perpendiculaires, dont une est parallèle à l'axe longitudinal de l'élément et l'autre est perpendiculaire à cet axe, cette partie étant traversée par un axe transversal sur lequel est monté pivotant un doigt allongé, ce doigt pouvant pivoter entre une position parallèle à l'axe longitudinal dudit élément et une position perpendiculaire à cet axe, en étant, dans l'une et l'autre de ces positions, en butée contre l'une ou l'autre des parois précitées, et
- l'élément présentant la surface glénoïde comprend une cavité axiale dans laquelle ce doigt est engagé et peut coulisser.

La forme de ladite surface condylienne permet le déplacement des os l'un par rapport à l'autre au cours du mouvement de flexion/extension de l'articulation, et le coulissement du doigt dans la cavité permet de guider ce déplacement tout en assurant un parfait contrôle de la laxité latérale de l'articulation.

Avantageusement, le doigt et la cavité présentent une section non circulaire et cette cavité est ajustée à ce doigt, de sorte que tout pivotement longitudinal d'un élément par rapport à l'autre est empêché.

De préférence, la tige d'ancrage de chaque élément présente au moins un méplat assurant son calage en rotation par rapport à l'os.

Chaque tige d'ancrage présente avantageusement un traitement de surface permettant de rendre sa surface poreuse, en vue d'un ancrage par ostéogenèse. Ce traitement de surface peut notamment être celui connu sous la dénomination "POROCOAT".

L'invention concerne également un ensemble d'éléments permettant de constituer une prothèse telle que précitée, comprenant :
- au moins deux éléments à surface condylienne, dont les tiges d'ancrage présentent des sections différentes, et
- au moins deux éléments à surface glénoïde, dont les longueurs et dimensions diffèrent,
ces différents éléments présentant des doigts et des cavités de mêmes dimensions d'un élément à l'autre, de sorte que tous les éléments à surface condylienne sont compatibles avec tous les éléments à surface glénoïde et permettent de constituer une prothèse adaptée aux différentes dimensions d'articulations susceptibles d'être remplacées.

Pour sa bonne compréhension, l'invention est à nouveau décrite ci-dessous en référence au dessin schématique annexé représentant, à titre d'exemple non limitatif, une forme de réalisation préférée d'un ensemble d'éléments permettant de constituer la prothèse d'articulation métacarpo-phalangienne ou inter-phalangienne qu'elle concerne.
La figure 1 est une vue en perspective des d'éléments composant cet ensemble ;
la figure 2 est une vue en perspective de l'un de ces éléments, avant montage, et
les figures 3 et 4 sont des vues en coupe longitudinale d'une prothèse, respectivement en position de flexion et d'extension.

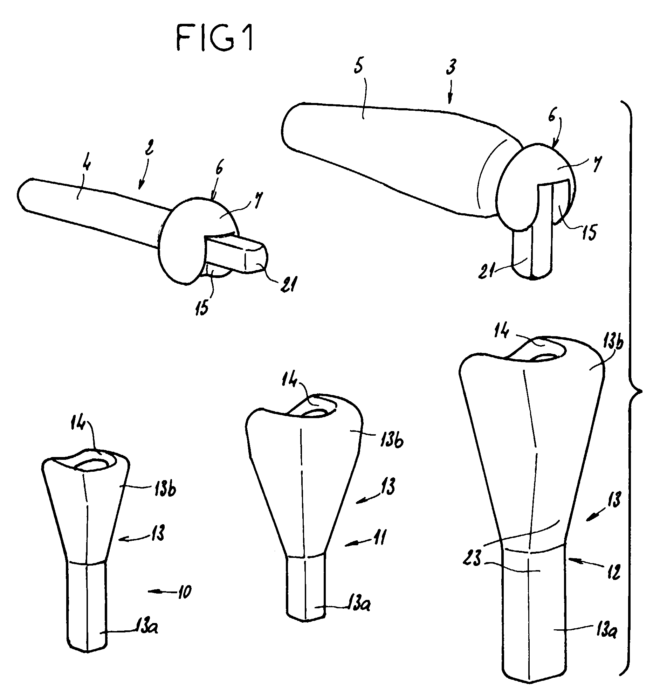

La figure 1 représente un ensemble
- de deux éléments 2, 3, comprenant chacun une tige médullaire d'ancrage 4, 5 et une tête arrondie 6 formant une surface condylienne 7, et de
- trois éléments 10, 11, 12, comprenant chacun une partie médullaire d'ancrage 13 et une cavité formant une surface glénoïde 14.

Cette surface glénoïde 14 est apte à coopérer avec la surface condylienne 7 de chaque élément 2, 3 pour former une prothèse d'articulation métacarpo-phalangienne ou inter-phalangienne.

Les tiges d'ancrage 4, 5 ont des sections différentes et les éléments 10, 11, 12 ont des longueurs et dimensions différentes, de sorte que des prothèses de dimensions différentes, adaptées aux différentes dimensions des articulations d'une main susceptibles d'être remplacées, peuvent être obtenues par coopération de l'un ou l'autre des éléments 2, 3 avec l'un ou l'autre des éléments 10, 11, 12.

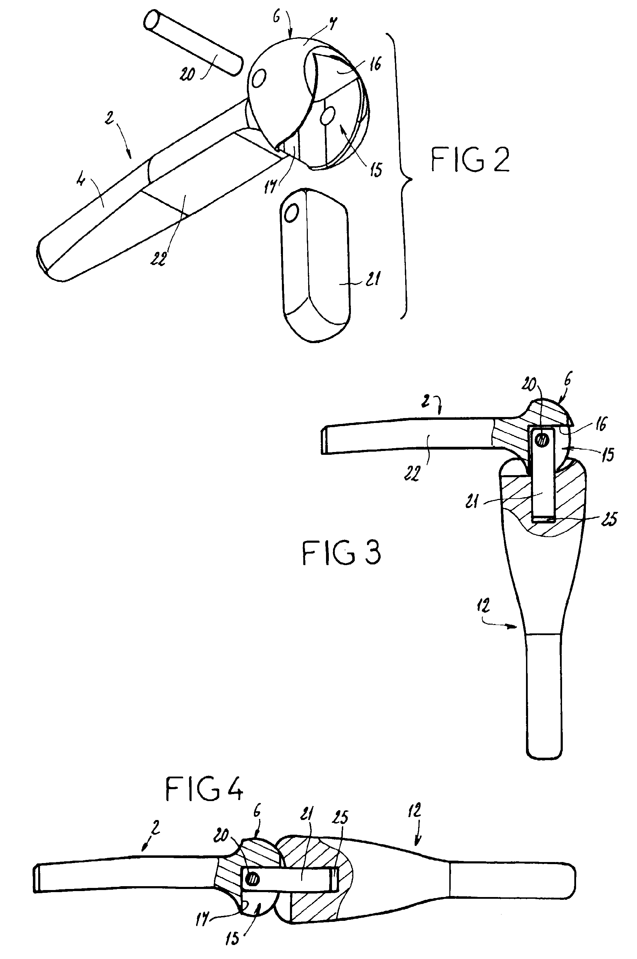

Ainsi que cela apparaît plus particulièrement aux figures 2 à 4, la surface condylienne 7 de chacun des éléments 2, 3 a un rayon de courbure non constant. Ce rayon augmente en direction de la périphérie de cette surface 7, de sorte que cette dernière présente la forme d'une portion de surface sphérique aplatie.

La tête 6 de chaque élément 2, 3 comprend une cavité centrale 15 délimitée par deux faces perpendiculaires 16, 17, dont une est parallèle à l'axe longitudinal de l'élément 2, 3 et l'autre est perpendiculaire à cet axe.

La tête 6 est traversée par un axe transversal 20, fixé à elle, sur lequel est monté pivotant un doigt allongé 21. Ce doigt 21 peut pivoter autour de l'axe 20 entre une position parallèle à l'axe longitudinal dudit élément 2, 3, montrée à la figure 4, et une position perpendiculaire à cet axe, montrée à la figure 3, en étant, dans l'une et l'autre de ces positions, en butée contre l'une ou l'autre des parois 16, 17.

Le doigt 21 présente une forme sensiblement parallélépipédique.

La tige 4 a une forme effilée comparativement à la tige 5, celle-ci étant plus massive et adaptée à des canaux médullaires de diamètres plus importants. Chacune de ces tiges 4, 5 présente un méplat 22 assurant son calage en rotation par rapport à l'os.

Chacun des éléments 10 à 12 a une portion médullaire effilée 13a et une portion métaphysaire évasée 13b, ces portions présentant des méplats 23 de calage par rapport à l'os.

Dans chaque portion 13b est aménagée une cavité axiale 25, ainsi que cela apparaît aux figures 3 et 4, en dessous de la cavité délimitant la surface 14. Ces différentes cavités 25 sont dimensionnées pour recevoir à coulissement le doigt 21 de l'un ou l'autre des éléments 2, 3, tout en étant ajustées à celui-ci.

La forme de la surface condylienne 7 permet le déplacement des os l'un par rapport à l'autre au cours du mouvement de flexion/extension de l'articulation, et le coulissement du doigt 21 dans la cavité 25 permet de guider ce déplacement tout en assurant un parfait contrôle de la laxité latérale de l'articulation.

## Revendications

1. Prothèse d'articulation métacarpo-phalangienne ou inter-phalangienne, comprenant deux éléments (2, 3, 10 à 12) destinés à être ancrés respectivement, au moyen de tiges d'ancrage (4, 5, 13), dans les canaux médullaires des os concernés, dont un (4, 5) présente une surface condylienne (7) et l'autre (10 à 12) une surface glénoïde (14), prothèse caractérisée en ce que :
- ladite surface condylienne (7) a une forme courbe mais avec un rayon de courbure non constant, qui augmente en direction de l'extrémité axiale de cette surface (7) située du côté opposé à la tige d'ancrage (4), de sorte que cette surface (7) présente la forme d'une portion de sphère aplatie, ladite extrémité axiale étant "palmaire", c'est-à-dire présentant un relativement faible rayon de courbure ;
- l'élément (2, 3) comprenant cette surface condylienne (7) comprend, au niveau de sa partie (6) qui forme cette même surface (7), une cavité centrale (15) délimitée par deux faces perpendiculaires (16, 17), dont une est parallèle à l'axe longitudinal de l'élément (2, 3) et l'autre est perpendiculaire à cet axe, cette partie (6) étant traversée par un axe transversal (20) sur lequel est monté pivotant un doigt allongé (21), ce doigt (21) pouvant pivoter entre une position parallèle à l'axe longitudinal dudit élément (2, 3) et une position perpendiculaire à cet axe, en étant, dans l'une et l'autre de ces positions, en butée contre l'une ou l'autre des parois précitées (16, 17), et
- l'élément (10 à 12) présentant la surface glénoïde (14) comprend une cavité axiale (25) dans laquelle ce doigt (21) est engagé et peut coulisser.

2. Prothèse selon la revendication 1, caractérisée en ce que le doigt (21) et la cavité (25) présentent une section non circulaire, et en ce que cette cavité (25) est ajustée à ce doigt (21), de sorte que tout pivotement longitudinal d'un élément (2, 3 ; 10 à 12) par rapport à l'autre est empêché.

3. Prothèse selon la revendication 1 ou la revendication 2, caractérisée en ce que la tige d'ancrage (4, 5, 13) de chaque élément présente au moins un méplat (22, 23) assurant son calage en rotation par rapport à l'os.

4. Prothèse selon l'une des revendications 1 à 3, caractérisée en ce que chaque tige d'ancrage (4, 5, 13) présente un traitement de surface permettant de rendre sa surface poreuse, en vue d'un ancrage par ostéogenèse.

5. Prothèse selon la revendication 4, caractérisée en ce que ledit traitement de surface est celui connu sous la dénomination "POROCOAT".

6. Ensemble d'éléments permettant de constituer une prothèse selon l'une des revendications 1 à 5, caractérisé en ce qu'il comprend :
- au moins deux éléments (2, 3) à surface condylienne (7), dont les tiges d'ancrage (4, 5) présentent des sections différentes, et
- au moins deux éléments (10 à 12) à surface glénoïde, dont les longueurs et dimensions diffèrent,
ces différents éléments (2, 3 ; 10 à 12) présentant des doigts (21) et des cavités (25) de mêmes dimensions d'un élément à l'autre, de sorte que tous les éléments à surface condylienne sont compatibles avec tous les éléments à surface glénoïde et permettent de constituer une prothèse adaptée aux différentes dimensions d'articulations susceptibles d'être remplacées.
